# EUROPÄISCHE PATENTSCHRIFT

(11) **EP 2 225 410 B1**
(45) Veröffentlichungstag und Bekanntmachung des Hinweises auf die Patenterteilung: **01.01.2020**
(21) Anmeldenummer: 08850754.6
(22) Anmeldetag: 11.11.2008
(51) Int. Cl.: D01F 2/00, D01F 1/10, C08L 1/02, C09K 5/06, F28D 20/00

(54) **VERFAHREN ZUR HERSTELLUNG VON CELLULOSISCHEN FORMKÖRPERN, CELLULOSISCHER FORMKÖRPER UND DESSEN VERWENDUNG**
PROCESS FOR PRODUCING CELLULOSIC SHAPED ARTICLES, CELLULOSIC SHAPED ARTICLES AND THE USE THEREOF
PROCÉDÉ DE FABRICATION DE CORPS MOULÉS EN CELLULOSE, CORPS MOULÉ EN CELLULOSE ET SON PROCÉDÉ D'UTILISATION

(30) Priorität: 14.11.2007 DE 102007054702
(43) Veröffentlichungstag der Anmeldung: 08.09.2010
(73) Patentinhaber: Smartpolymer GmbH, 07407 Rudolstadt (DE)
(72) Erfinder: KOLBE, Axel, 07924 Neuendorf (DE); MARKWITZ, Hardy, 07407 Rudolstadt (DE); RIEDE, Sabine, 07407 Uhlstädt-Kirchhasel (DE); KRIEG, Marcus, 99423 Weimar (DE)
(74) Vertreter: Plate, Jürgen
(86) Internationale Anmeldenummer: PCT/EP2008/009497
(87) Internationale Veröffentlichungsnummer: WO 2009/062657

(56) Entgegenhaltungen:
- EP-A- 1 174 036
- WO-A-99/31141
- WO-A-2005/017247
- WO-A-2008/040320
- US-A- 5 565 132
- DEGUSSA TECHNICAL DATASHEET: "AEROSIL R972 HYDROPHOBIC FUMED SILICA (PRODUCT INFORMATION)" ANNOUNCEMENT DEGUSSA, XX, XX, 1. Mai 2005 (2005-05-01), Seiten 1-2, XP002395339
- XINGXIANG ZHANG: "Heat-storage and thermo-regulated textiles and clothing" SMART FIBRES, FABRICS AND CLOTHING, WOODHEAD PUBLISHING, Bd. Capter 3, 1. Januar 2001 (2001-01-01), Seiten 34-57, XP002492889 ISBN: 978-1-85573-546-0

## Beschreibung

Die Erfindung betrifft ein Verfahren zur Herstellung von cellulosischen Formkörpern mit Einschlüssen mindestens einer unpolaren organischen Verbindung nach dem Trocken-Nass-Extrusionsverfahren, einen cellulosischen Formkörper und dessen Verwendung.

Es ist bekannt, dass die Wärmespeicherkapazität von textilen Fasern und Formkörpern erhöht werden kann, wenn das formgebende Polymer mit einem organischen Phasenwechselmaterial kombiniert wird, das durch Schmelz/Erstarrungsübergang, Konformationsübergang oder Entorientierung/ Kristallisation Energie mit der Umgebung austauschen kann. Die Höhe des Energieaustauschs und der wirksame Temperaturbereich korrelieren mit der chemischen Struktur, der Änderung der physikalischen Enthalpie und der Konzentration des Phasenwechselmaterials. Entscheidend ist in erster Linie, dass der Energieaustauscheffekt in der Faser durch die molekulare Nahorientierung des Phasenwechselmaterials in oder auf dem Formkörper erhalten bleibt. Bekannt sind folgende Lösungen:
In erster Linie werden Phasenwechselmaterialien mit einer organischen Polymerschicht verkapselt und anschließend die Kapseln in eine Polymerfaser ein- oder auf ein Gewebe aufgebracht (z. B. EP 1 658 395 = US 2006/0279017). Mikroverkapselte Phasenwechselmaterialien werden auch in den Beispielen gemäß der WO 2005/017247 bei der Herstellung von Cellulosefasern mit thermoregulativen Eigenschaften nach dem Lyocell-Verfahren eingesetzt. Als nachteilig erweist sich dabei, dass die Verkapselung des Phasenwechselmaterials von der Formgebung, bzw. von der Verarbeitung getrennt erfolgt. Zwangsläufig ist ein Kompromiss zwischen verfügbaren Kapselchargen hinsichtlich Material und Eignung für das Formgebungsverfahren erforderlich. Bei Trocken-Nass-Extrusionsverfahren werden an Mikrokapseln unter anderem Anforderungen wie Feinheit und Korngrößenverteilung, mechanische und chemische Stabilität, Eignung des Phasenwechselmaterials für das Einsatzgebiet, Verfügbarkeit und Preis, gestellt.

Weiterhin können Phasenwechselmaterialien in eine Polyolefinmatrix oder eine Polymersuspension eingearbeitet werden. Bekannt ist beispielsweise die Herstellung von schmelzegesponnenen Polyolefinfasern, die Phasenwechselmaterialien mit einem Schmelzpunkt von 15 bis 65 °C enthalten (US 5 885 475).

Das direkte Einarbeiten eines Phasenwechselmaterials (z.B. eines Polyethylenglykols) in eine Hohlfaser ist in der US 4 908 238 beschrieben. Hier wurde jedoch auf eine Stabilisierung des Phasenwechselmaterials im Formkörper verzichtet. Von der Struktur her ähnelt es einer Mikrosandwich-Konstruktion. Einfache Sandwichstrukturen sind z.B. in der US 2003/124278 offenbart.

Gemäß einer besonderen Ausführungsform der WO 03/027365 (= EP 1 430 169) soll es möglich sein, das PCM bei der Herstellung einer Cellulosefaser in Rohform einzumischen. Dabei kann jedoch keine permanente Bindung des PCMs zum Matrixmaterial (Cellulose) entstehen, und es ist auch nicht möglich, aus einer Mischung von PCM und gelöster Cellulose eine Faser zu erspinnen.

Es besteht ein Interesse, Wirkstoffe aus einem Gewebe oder einer Cellulosefaser freizusetzen. Es ist auch bekannt, verkapselte, Wirkstoff enthaltende Materialien an der Oberfläche von Fasern zu verankern (WO 01/73188) oder sie darin einzubringen (WO 2006/066291). Die Möglichkeit Riechstoffe und Wirkstoffe als Mikrokapseln herzustellen, ist z.B. in der EP 1 243 326 beschrieben. Wiederum erweist sich die Mikrokapsel durch die begrenzte Verfügbarkeit als Nachteil für eine großtechnische Anwendung, da die Verkapselung separat von der Formgebung erfolgt.

Aus der Literatur sind keine Ansätze bekannt, wie sich die Erzeugung von permanenten unpolaren organischen Mikroeinschlüssen in ein hydrophiles netzwerkbildendes Polymer, wie Cellulose, durch Zugabe der Rohstoffe (Lösungsmittel, Cellulose, unpolare organische Verbindungen und Gemische, Verdickungsmittel und Phasenvermittler) zur Spinnlösung und anschließender Formgebung in einem Verfahren realisieren ließe. Bisher wurde auch nicht beschrieben, dass organische Verbindungen, die in den unpolaren organischen Verbindungen und Gemischen gelöst oder suspendiert werden können, als Modifikatoren (Veränderung des Schmelzbereichs von Phasenwechselmaterialien durch z.B. Schmelzpunkterniedrigung) oder freisetzbare Wirkstoffe genutzt werden können, wenn sie als permanente, unpolare organische Mikroeinlagerungen in ein hydrophiles netzwerk-bildendes Polymer, wie Cellulose, eingebracht werden würden.

Bekannt war lediglich der Einbau von nanoskaligen Wirkstoffen in Pulverform bzw. von Carbon-Nanotubes (WO 2004/081267). Eine Lehre in Bezug auf die Einarbeitung von lipophilen Substanzen in eine polare Celluloselösung kann daraus nicht abgeleitet werden.

Ausgehend von einem Stand der Technik, wie er in der WO 2006/066291 dargestellt ist, lag der Erfindung dementsprechend die Aufgabe zugrunde, ein direktes Verfahren zur Herstellung von cellulosischen Formkörpern mit Einschlüssen unpolarer organischer Verbindungen und Gemischen unter Nutzung der direkten Einarbeitung dieser organischen, unpolaren Verbindungen und Gemische zu entwickeln. Gegebenenfalls sollen diese cellulosischen Formkörper mit Einschlüssen unpolarer organischer Verbindungen mit weiteren funktionellen Zusätzen ausgestattet werden, die besonders an oder nahe der Oberfläche der Einschlüsse angereichert sind.

Außerdem ist im Rahmen der Aufgabe, ein Verfahren zu entwickeln, bei dem Wirkstoffe in cellulosischen Formkörpern gelöst und/oder gespeichert und kontrolliert über einen längeren Zeitraum an die Umgebung abgegeben werden können.

Diese Aufgabe wird erfindungsgemäß dadurch gelöst, dass
a1) eine Emulsion mit mindestens einer unpolaren organischen Verbindung in einer Lösung von Cellulose in einem Lösungsmittel aufbereitet und durch Zugabe von mindestens einem hydrophoben viskositätssteigernden Mittel stabilisiert wird, das die Viskosität der unpolaren organischen Verbindung steigert, wobei als Lösungsmittel eine ionische Flüssigkeit eingesetzt wird,
   oder
a2) der Emulsion nanoskalige, flächige und/oder längliche, hydrophobierte Partikel, zum Beispiel Schichtsilikate, Nanotubes oder Nanofilamente, zugesetzt werden, die die tröpfchenartigen Einschlüsse der unpolaren organischen Verbindung/en umgeben und eine Suspension bilden,
   und
b) die Cellulose rekristallisiert wird, wodurch Formkörper mit einer Cellulosematrix erhalten werden, in der die unpolare/n organische/n Verbindung/en dispers eingelagert ist/sind,
wobei die cellulosischen Formkörper Fasern sind und wobei die nanoskaligen, flächigen und/oder länglichen hydrophobierten Partikel modifizierte Schichtsilikate umfassen.

Bei dem Verfahren wird eine cellulosische Form- und Spinnlösung, die durch Lösen von Cellulose in einem geeigneten Lösungsmittel hergestellt werden kann, mit einem unpolaren organischen Material versetzt, die Viskosität des unpolaren Materials erhöht, damit es sich in der Cellulose-Lösung emulgieren läßt, und die dispergierte Phase unpolaren Materials stabilisiert. Werden flächige und/oder längliche nanoskalige Partikel zugesetzt, wie hydrophobierte Schichtmineralien, z.B. Schichtsilikate und Bentonite, die in der Spinnmasse exfoliert werden, wird die dispergierte Phase mit einer Schicht aus diesen Nanopartikeln umgeben.

Anstelle von Cellulose oder zusätzlich dazu können beliebige Polysaccharide in natürlicher oder synthetisch hergestellter Form eingesetzt werden und/oder auch Mischungen von Polysacchariden. Beispiele sind Holzzellulose, Stärke, Jute, Flachs, Baumwoll-Linters, Chitosan oder Mischungen daraus.

Geeignete Lösungsmittel sind ionische Flüssigkeiten, vorzugsweise Ethylmethylimidazoliumacetat. Es hat sich gezeigt, dass auch andere ionische Flüssigkeiten als Lösungsmittel für dieses Verfahren geeignet sind, zum Beispiel 1-Butyl-3-methyl-imidazoliumchlorid (BMIMCl), 1-Ethyl-3-methyl-imidazoliumchlorid (EMIMCl), 1-Butyl-3-methyl-imidazoliumacetat (BMIMAc) und 1-Ethyl-3-methylimid-azoliumacetat (BMIMAc), 1-Alkyl-3-methyl-imidazoliumsalze.

Beim erfindungsgemäßen Verfahren erfolgt die Herstellung und das Verspinnen einer physikalischen Lösung der Cellulose ohne deren Derivatisierung, indem eine Emulsion mit mindestens einer unpolaren organischen Verbindung in einer Lösung von Cellulose in einer ionischen Flüssigkeit hergestellt und durch Zugabe von hydrophoben viskositätssteigernden Mitteln (Verdickungsmitteln) stabilisiert wird, sowie eine Suspension geformt und die Cellulose rekristallisiert wird, wodurch Formkörper mit einer Cellulosematrix erhalten werden, in der die unpolare organische Verbindung dispers eingelagert ist. Gegebenenfalls können der Emulsion nanoskalige, flächige und/oder längliche, hydrophobierte Partikel zugesetzt werden, die die eingelagerten Tröpfchen der unpolaren organischen Verbindung umhüllen, was zu einer weiteren Stabilisierung des unpolaren Materials führt.

Bei der Arbeit mit ionischen Flüssigkeiten ist von besonderem Interesse, dass die Verarbeitung von geeigneten Cellulose/Salz-Lösungen bei Temperaturen unterhalb von 90 °C erfolgt, da die Spinnmasse im Gegensatz zu Cellulose/Aminoxid- Lösungen nicht erstarrt und damit formbar in einem breiteren Temperaturfenster von Raumtemperatur bis 120 °C bleibt. Zum Beispiel können damit auch unpolare Materialien mit einem signifikanten Dampfdruck bei 90 °C verarbeitet werden.

Die unpolare organische Verbindung ist bevorzugt ein Kohlenwasserstoff, ein Wachs, Bienenwachs, ein Öl, eine Fettsäure, ein Fettsäureester, Stearinanhydrid, ein langkettiger Alkohol oder eine beliebige Mischung davon. Sie hat allgemein einen Schmelzpunkt von weniger als 100 °C und bevorzugt einen Schmelzpunkt im Bereich von 0 bis 40 °C. Das gilt auch für die Mischungen.

Eines der bevorzugten hydrophoben viskositätssteigernden Mittel ist eine hydrophobierte nanoskalige pyrogene Kieselsäure. Es erhöht die Viskosität der unpolaren organischen Verbindung(en) so weit, dass sie in der cellulosischen Form- und Spinnlösung emulgiert werden können. Weitere geeignete Verdickungsmittel sind Polymere mit olefinischen und aromatischen Anteilen, wie zum Beispiel Styrol-Butadien-Blockpolymere oder kurzkettige Polyethylentypen oder phosphorhaltige Ester. Dabei handelt es sich hauptsächlich um bizyklische phosphorhaltige Ester, die zusätzlich einen Flammschutzeffekt bieten. Auch hydrophobierte, nanoskalige flächige und/ oder längliche Partikel können als Verdickungsmittel eingesetzt werden. Überraschenderweise reicht dafür ein Anteil an Verdickungsmitteln von 1 bis 50 Gew.-%, bevorzugt von 5 bis 20 Gew.-%, aus, bezogen auf das Gewicht der Cellulose, um die im Vergleich zu Kohlenwasserstoff-in-Wasser-Emulsionen viel größeren Dichte- und Viskositätsunterschiede einer Emulsion von Kohlenwasserstoffen in einer cellulosischen Form- und Spinnlösung zu überbrücken. Die nanoskalige pyrogene Kieselsäure besteht allgemein aus Partikeln mit einem mittleren Durchmesser von 30 bis 200 nm, bevorzugt von 40 bis 100 nm.

Für das erfindungsgemäße Verfahren geeignete hydrophobierte, nanoskalige, pyrogene Kieselsäuren sind bekannt. Im Stand der Technik dienen sie zur Verdickung von Lösungen (EP 0 745 372) sowie der Stabilisierung von Wasser-in-ÖI oder Öl-in-Wasser-Emulsionen gegen Separation der dispersen Phase durch Anlagerung der pyrogenen Kieselsäure an der Grenzfläche Öl/Wasser (DE 10 2004 014 704). Sie können für "controlled release"-Systeme verwendet werden.

Die nanoskaligen, flächigen, hydrophobierten Partikel werden allgemein ebenfalls in einem Anteil von 1 bis 50 Gew.-%, bevorzugt von 2 bis 20 Gew.-%, besonders bevorzugt von 5 bis 12 Gew.-%, jeweils bezogen auf das Gewicht der Cellulose, eingesetzt. Vorzugsweise handelt es sich dabei um modifizierte Schichtsilikate, z.B. hydrophobiertes Bentonit. Die Partikel haben allgemein eine Länge und Breite von etwa 200 bis 1.000 nm und eine Dicke von etwa 1 bis 4 nm. Das Verhältnis von Länge und Breite zu Dicke (aspect ratio) beträgt vorzugsweise etwa 150 bis 1.000, bevorzugt von 200 bis 500. Ebenso können hydrophobierte längliche nanoskalige Partikel eingesetzt werden, zum Beispiel Carbon Nanotubes oder Carbon Nanofilamente. Nanotubes haben allgemein einen Durchmesser <1 bis 30 nm, Nanofilamente von ca. 150-300 nm. Die Länge beträgt bis zu mehreren Millimetern.

Die nanoskaligen Partikel umgeben die organischen Materialmikrophasen mit einer Schicht nanodisperser Strukturen. Die Partikel haben die überraschende Eigenschaft, dass sie die Emulsion während der Formgebung stabilisieren und anschließend als Phasenvermittler zwischen Cellulosematrix und eingeschlossenem unpolaren organischen Verbindungen fungieren.

Als "nanoskalig" werden im Zusammenhang mit der vorliegenden Erfindung Objekte bezeichnet, die in mindestens einer Dimension eine Größe von 1 bis 100 nm aufweisen, wie in der technischen Norm ISO/TS 27687 ausgeführt.

Die eingeschlossenen unpolaren organischen Verbindungen lassen sich auch mit Wirkstoffen beladen. Dabei handelt es sich um unpolare Wirkstoffe, die mit den unpolaren organischen Verbindungen Lösungen oder Suspensionen bilden. Bei den Wirkstoffen handelt es sich bevorzugt um Pflanzenprodukte, wie Jojobaöl, Monoi-Öl, Nachtkerzen-Öl, Avocado-Öl, Kakaobutter, ätherische Pflanzenextrakte oder unpolare Pflanzenauszüge, um fettlösliche Vitamine, wie Vitamin A, D und E, oder um Insektizide, wie Pyrethroide, speziell Permethrin, oder um Repellents. Die Konzentration an Wirkstoff(en) kann von 0,001 g pro kg bis zu 500 g und mehr, bevorzugt von 50 bis 150 g pro kg Formkörper betragen. Die Wirkstoffe können über einen längeren Zeitraum kontrolliert an die Umgebung abgegeben werden. Dieser Effekt läßt sich z.B. mit der Waschpermanenz der Funktionsfaser belegen.

Die hydrophobierten nanoskaligen Partikel innerhalb des cellulosischen Formkörpers können ebenfalls mit unpolaren und anderen organischen oder anorganischen Substanzen beladen werden. Solche organische oder anorganische Substanzen umfassen zum Beispiel Farbstoffe, Pigmente, Flammschutzmittel, Weichmacher, lumineszierende Substanzen, UV-Absorber, elektrisch oder magnetisch leitende Substanzen, Mattierungsmittel, Geruchsstoffe, antibakterielleWirkstoffe, Fungizide und andere funktionelle Zusätze. Durch schwache zwischenmolekulare Wechselwirkungen werden diese Moleküle reversibel adsorbiert. Es zeigt sich, dass überschüssige nanoskalige Partikel bevorzugt an der Oberfläche des Formkörpers bzw. in der Nähe der Oberfläche der Einschlüsse sich anreichern. Dies eröffnet eine weitere Möglichkeit, dem Formkörper zusätzliche funktionale Eigenschaften zu verleihen. Durch die Quellfähigkeit der Cellulose können die Formkörper auch nach dem Fertigungsprozess mit unpolaren und schwach polaren Materialien beladen werden, indem unpolare und schwach polare Materialien aus einer wässrigen Phase an die Oberfläche der Nanopartikel wandern und dort reversibel adsorptiv gebunden werden. Diese Vorgangsweise ist besonders gut für Substanzen geeignet, die ihre Wirkung über die Gasphase entfalten, wie Repellents gegen Insekten, Geruchsstoffe aller Art oder medizinische Wirksubstanzen. Weitere Zusätze in den Formkörpern können sein: Farbstoffe, UV-Stabilisatoren, bakterizide Substanzen, Flammschutzmittel, Antistatika, Vernetzungsmittel, Weichmacher, Katalysatoren.

Durch die Zugabe von unpolaren organischen Verbindungen und Gemischen in einer Konzentration kleiner 200 % (w/w), bezogen auf das Gewicht der in der Spinnlösung gelösten Cellulose, enthalten die Formkörper weniger als 66 % (w/w) an unpolaren organischen Substanzen oder Gemischen.

Das erfindungsgemäße Verfahren führt zu cellulosischen Formkörpern, die gegenüber unmodifizierten Cellulosefasern eine wesentlich gesteigerte Speicherkapazität für Wärme und/oder unpolare Wirksubstanzen aufweisen und deren Wirkung mit weiteren Funktionalitäten kombiniert sein kann.

Zusätzlich kann der Schmelzpunkt der Phasenwechselmaterialien durch Abmischen mit anderen organischen Verbindungen erniedrigt und damit auf den jeweils gewünschten Wert eingestellt werden. Die unpolaren organischen Verbindung(en) eignen sich ferner als Lösungsmittel und/oder Speichermedium für unpolare organische Wirkstoffe. Aus den Einschlüssen in den cellulosischen Formkörpern können die Wirkstoffe kontrolliert freigesetzt werden. Es kann auch der gegenteilige Effekt genutzt werden, bei dem die Fasern mit den Einschlüssen aus unpolaren organischen Verbindungen gasförmige und/oder flüssige unpolare Verbindungen (Schadstoffe) absorbieren.

Die funktionale Wirkung beruht auf dem physikalischen Effekt der Wärmespeicherung und/oder auf der gleichmäßigen und feindosierbare Speicherung und Freisetzung von unpolaren Wirkstoffen, Pflanzenextrakten und dgl. aus dem Faserinneren. Durch geeignete Wahl des unpolaren Anteils sind nach diesem Verfahren auch Fasern herstellbar, die als Absorptionsmedium für flüssige oder gasförmige, unpolare Substanzen dienen können. Weitere funktionelle Wirkungsweisen lassen sich durch Auswahl spezieller funktioneller Verdickungsmittel bzw. flächiger nanoskaliger Zusatzmittel, beladen mit funktionellen Wirkstoffen, erzielen.

Mit diesem Verfahren lassen sich cellulosische Formkörper mit den bereits beschriebenen Effekten, wie erhöhte Wärmespeicherkapazität und "controlled-release"-Funktionen, viel effizienter und kostengünstiger herstellen, da Bulkmaterialien verarbeitbar sind und die konventionelle Kapselung und die Einarbeitung von Mikrokapseln entfällt. Das Verfahren gemäß der Erfindung ist variabel. So kann z.B. die Schmelzpunkterniedrigung von Gemischen genutzt werden, um ein industrielles Standard-Phasenwechselmaterial exakt an eine vorgegebene Anwendungstemperatur anzupassen bzw. den Schmelz-/Erstarrungsbereich zu verbreitern.

Die erfindungsgemäßen Formkörper in Form von Fasern lassen sich zu Textilien verarbeiten, die in der Bekleidungsindustrie, als technische Textilien und im Freizeitbereich Anwendung finden. Speziell die mit unpolaren Wirkstoffen versehenen Formkörper können auch für medizinische oder kosmetische Zwecke eingesetzt werden. Die Formkörper können ferner zur Herstellung von Spezialpapieren oder von Folien dienen, die mit Wirkstoffen beladen sind.

Die erfindungsgemäßen cellulosischen Formkörper weisen eine Cellulosematrix und darin dispergierte Einschlüsse auf, wobei die Einschlüsse eine oder mehrere mit einem hydrophoben Verdickungsmittel stabilisierte unpolare organische Verbindungen enthalten.

Die unpolaren organischen Verbindungen sind bevorzugt ausgewählt aus der Gruppe, umfassend Kohlenwasserstoffe, Wachse, Bienenwachse, Öle, Fettsäuren, Fettsäureester, Stearinanhydride und langkettige Alkohole, die jeweils einen Schmelzpunkt von weniger als 100 °C haben. Der Anteil der unpolaren organischen Verbindungen beträgt mehr als 10 Gew.-%, bevorzugt mehr als 30 Gew.-%, und besonders bevorzugt mehr als 40 Gew.-%, bezogen auf das Gewicht der Cellulose.

Eines der hydrophoben Verdickungsmittel besteht aus nanoskaligen Partikeln, bevorzugt aus hydrophobierter nanoskaliger pyrogener Kieselsäure, und ist in einer Menge von 1 bis 50 Gew.-%, bezogen auf das Gewicht der Cellulose, enthalten.

Die Einschlüsse können zudem einen oder mehrere Wirkstoffe aus der Gruppe, umfassend Pflanzenprodukte, Jojobaöl, Monoi-Öl, Nachtkerzen-Öl, Avocado-Öl, Kakaobutter, ätherische Pflanzenextrakte, unpolare Pflanzenauszüge, fettlösliche Vitamine, Vitamin A, D und E, Insektizide, Pyrethroide, Permethrin und Repellents, enthalten. Die Wirkstoffe sind in einer Menge von bis zu 50 Gew.-%, bezogen auf das Gewicht des cellulosischen Formkörpers, enthalten.

In einer besonderen Ausführungsform enthält der cellulosische Formkörper ein Barrierematerial aus nanoskaligen schichtförmigen Partikeln und/ oder nanoskaligen länglichen Partikeln, mittels welcher die unpolaren organischen Verbindungen in den Einschlüssen zurückgehalten und Wirkstoffe in kontrollierter Weise freigesetzt werden. Der Anteil des Barrierematerials beträgt 1 bis 50 Gew.-%, bezogen auf das Gewicht der Cellulose.

In weiteren Ausführungsformen weist der cellulosische Formköper in einem Temperaturbereich von 15 bis 45 °C eine spezifische latente Wärme von größer 20 J/g, bevorzugt von größer 30 J/g und besonders bevorzugt von größer 50 J/g auf.

Im Folgenden wird die Erfindung anhand von zwei schematischen Figuren näher erläutert.

Es zeigen:
- Fig. 1: eine schematische Ansicht einer cellulosischen Faser und
- Fig. 2: eine Schnittansicht durch einen einzelnen Einschluss und seine Umgebung in der Faser nach Fig. 1.

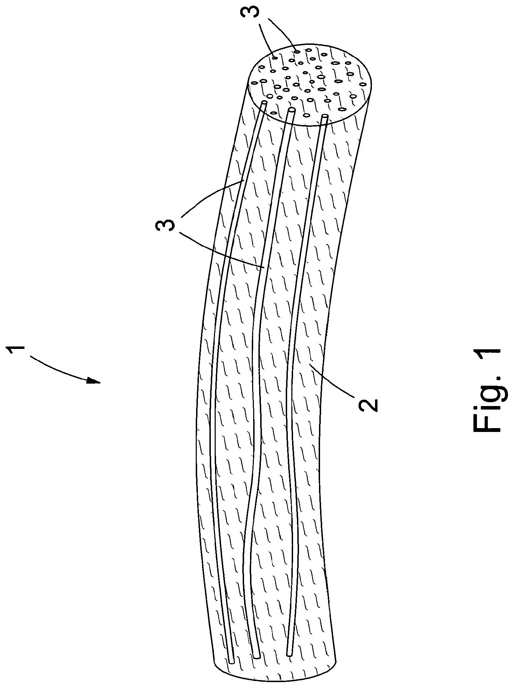

In Fig. 1 ist eine Faser 1 mit Cellulosematrix 2 und darin dispergierten Einschlüssen 3 dargestellt. Die Einschlüsse 3 enthalten eine oder mehrere unpolare organische Verbindungen, die mit zumindest einem hydrophoben Verdickungsmittel stabilisiert sind.

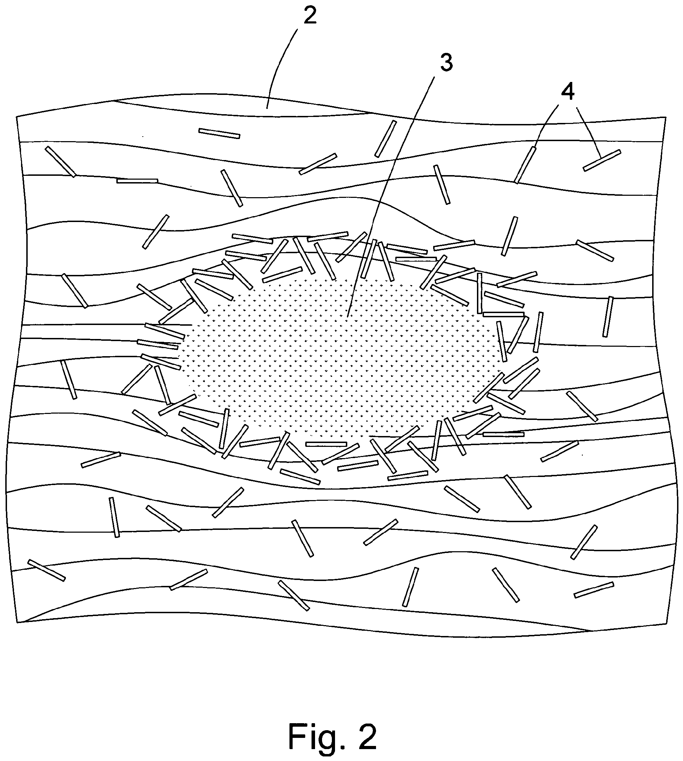

Details der Einschlüsse 3 und der sie umgebenden Cellulosematrix sind in Fig. 2 in schematischer Weise dargestellt. In der Cellulosematrix 2 ist ein Barrierematerial 4 aus nanoskaligen schichtförmigen Partikel dispergiert. Insbesondere liegen die schichtförmigen Partikel separat bzw. exfoliert in der Cellulosematrix 2 vor. Um die Einschlüsse 3 herum ist die Dichte des Barrierematerials 4 gegenüber seiner mittleren Dichte in der Cellulosematrix 2 erhöht. Dementsprechend sind die Einschlüsse 3 von einer Zone des Barrierematerials umgeben, durch welche die unpolaren organischen Verbindungen und ggf. darin enthaltene Wirkstoffe nicht bzw. nur auf verschlungenen Pfaden ("tortuous path") in die Cellulosematrix 2 eintreten können. Durch geeignete Wahl und Dosierung des Barrierematerials 4 kann die Permeabilität für Wirkstoffe in gezielter Weise eingestellt werden ("controlled release system").

## Patentansprüche

1. Verfahren zur Herstellung von cellulosischen Formkörpern mit Einschlüssen mindestens einer unpolaren organischen Verbindung nach dem Trocken-Nass-Extrusionsverfahren, **dadurch gekennzeichnet, dass**
a1) eine Emulsion mit mindestens einer unpolaren organischen Verbindung in einer Lösung von Cellulose in einem Lösungsmittel aufbereitet und durch Zugabe von mindestens einem hydrophoben Mittel stabilisiert wird, das die Viskosität der unpolaren organischen Verbindung steigert, wobei als Lösungsmittel eine ionische Flüssigkeit eingesetzt wird,
oder
a2) der Emulsion zur Stabilisierung nanoskalige, flächige und/oder längliche, hydrophobierte Partikel zugesetzt werden, die die tröpfchenartigen Einschlüsse der unpolaren organischen Verbindung/en umgeben und eine Suspension bilden, und
b) die Cellulose rekristallisiert wird, wodurch Formkörper mit einer Cellulosematrix erhalten werden, in der die unpolare/n organische/n Verbindung/en dispers eingelagert ist/sind,
wobei die cellulosischen Formkörper Fasern sind und wobei die nanoskaligen, flächigen und/oder länglichen hydrophobierten Partikel modifizierte Schichtsilikate umfassen.

2. Verfahren nach Anspruch 1, **dadurch gekennzeichnet, dass** von den länglichen Partikeln Nanotubes oder Nanofilamente eingesetzt werden.

3. Verfahren nach Anspruch 1, **dadurch gekennzeichnet, dass** als hydrophobes Mittel nanoskalig strukturierte, pyrogene Kieselsäure, Polymere mit olefinischen und aromatischen Anteilen, bevorzugt Styrol-Butadien-Blockpolymere, oder phosphorhaltige Ester eingesetzt werden.

4. Verfahren nach Anspruch 2, **dadurch gekennzeichnet, dass** hydrophobe Mittel mit einem Anteil von 1 bis 50 Gew.-%, bevorzugt 1 bis 30 Gew.-%, bezogen auf das Gewicht der Cellulose, eingesetzt werden.

5. Verfahren nach Anspruch 1, **dadurch gekennzeichnet, dass** sowohl dem/den hydrophoben Mittel/n als auch den flächigen Nanopartikeln Farbstoffe, UV-Stabilisatoren, bakterizide Substanzen, Flammschutzmittel, Antistatika, Vernetzungsmittel, Weichmacher und/oder Katalysatoren zugesetzt werden.

6. Verfahren nach Anspruch 1, **dadurch gekennzeichnet, dass** als unpolare organische Verbindungen Kohlenwasserstoffe, Wachse, Bienenwachse, Öle, Fettsäuren, Fettsäureester, Stearinanhydride, langkettige Alkohole oder Mischungen davon, jeweils mit einem Schmelzpunkt von weniger als 100 °C, eingesetzt werden.

7. Verfahren nach Anspruch 1, **dadurch gekennzeichnet, dass** die unpolare/n organische/n Verbindung/en mit anderen organischen Substanzen abgemischt wird/werden, die den Schmelzpunkt der unpolare/n organische/n Verbindung/en erniedrigen.

8. Verfahren nach Anspruch 1, **dadurch gekennzeichnet, dass** die Einschlüsse der unpolaren organischen Verbindung/en aus Lösungen oder Suspensionen von Wirkstoffen, vorzugsweise unpolaren Wirkstoffen in unpolaren organischen Lösungsmitteln erhalten werden, wobei als Wirkstoffe bevorzugt Pflanzenprodukte, wie Jojobaöl, Monoi-Öl, Nachtkerzen-Öl, Avocado-Öl, Kakaobutter, ätherische Pflanzenextrakte oder unpolare Pflanzenauszüge, fettlösliche Vitamine, wie Vitamin A, D und E, sowie Insektizide, Pyrethroide, bevorzugt Permethrin, oder Repellents eingesetzt werden, wobei die Konzentration an Wirkstoff/en 0,001 bis 500 g und mehr pro kg Formkörper beträgt.

9. Cellulosischer Formkörper, herstellbar nach einem Verfahren gemäß einem oder mehreren der Ansprüche 1 bis 8, mit einer Cellulosematrix und darin dispergierten Einschlüssen aus der/den unpolare/n organische/n Verbindung/en, der mindestens ein hydrophobes, viskositätssteigerndes Mittel enthält oder in dem die Einschlüsse von nanoskaligen flächigen und/oder länglichen hydrophobierten Partikeln als Barrierematerial umgeben sind.

10. Cellulosischer Formkörper nach Anspruch 9, **dadurch gekennzeichnet, dass** die unpolare/n organische/n Verbindung/en ausgewählt ist/sind aus der Gruppe, umfassend Kohlenwasserstoffe, Wachse, Bienenwachse, Öle, Fettsäuren, Fettsäureester, Stearinanhydride und langkettige Alkohole, die jeweils einen Schmelzpunkt von weniger als 100 °C aufweisen, oder daß der Anteil der unpolaren organischen Verbindung/en mehr als 10 Gew.-%, bevorzugt mehr als 30 Gew.-%, und besonders bevorzugt mehr als 40 Gew.-%, bezogen auf das Gewicht der Cellulose, beträgt, wobei die nanoskaligen Partikel bevorzugt aus hydrophobierter nanoskaliger pyrogener Kieselsäure bestehen.

11. Cellulosischer Formkörper nach Anspruch 9, **dadurch gekennzeichnet, dass** die Einschlüsse einen oder mehrere Wirkstoffe aus der Gruppe, umfassend Pflanzenprodukte, Jojobaöl, Monoi-Öl, Nachtkerzen-Öl, Avocado-Öl, Kakaobutter, ätherische Pflanzenextrakte, unpolare Pflanzenauszüge, fettlösliche Vitamine, Vitamin A, D und E, Insektizide, Pyrethroide, Permethrin und Repellents, enthalten, wobei die Wirkstoffe bevorzugt in einer Menge von bis zu 50 Gew.-%, bezogen auf das Gewicht des cellulosischen Formkörpers, enthalten sind.

12. Cellulosischer Formkörper nach Anspruch 9, **dadurch gekennzeichnet, dass** der Anteil des/der hydrophoben Mittel/s 1 bis 50 Gew.-%, bezogen auf das Gewicht der Cellulose, beträgt.

13. Cellulosischer Formkörper nach den Ansprüchen 9 und 11, **dadurch gekennzeichnet, dass** er ein Barrierematerial aus nanoskaligen Partikeln zur Rückhaltung der unpolaren organischen Verbindungen und zur kontrollierten Freisetzung der Wirkstoffe enthält, wobei der Anteil des Barrierematerials bevorzugt 1 bis 50 Gew.-%, bezogen auf das Gewicht der Cellulose, beträgt oder wobei das Barrierematerial bevorzugt nanoskalige hydrophobierte Schichtsilikate, nanoskaligen hydrophobierten Bentonit oder Nanotubes oder Nanofilamente umfasst.

14. Cellulosischer Formkörper nach Anspruch 9, **dadurch gekennzeichnet, dass** die Einschlüsse von einer Zone mit erhöhter Dichte des Barrierematerials umgeben sind.

15. Cellulosischer Formkörper nach Anspruch 9, **dadurch gekennzeichnet, dass** er eine Faser ist und dass nach einem Test gemäß DIN EN 26330 (1993) der Verlust einer oder mehrerer unpolarer organischer Verbindungen nach 20 Wäschen weniger als 20 Gew.-%, bezogen auf die ursprünglich in der Faser enthaltene Menge der jeweiligen organischen Verbindung, beträgt.

16. Cellulosischer Formkörper nach Anspruch 9, **dadurch gekennzeichnet, dass** er in einem Temperaturbereich von 15 bis 45 °C eine spezifische latente Wärme von größer 20 J/g, bevorzugt von größer 30 J/g und besonders bevorzugt von größer 50 J/g aufweist.

17. Verwendung cellulosischer Formkörper nach Anspruch 9 in textilen Flächengebilden, gegebenenfalls unter Abmischung mit anderen textilen Fasern, zur Herstellung von Spezialpapieren.

## Claims

1. Process for producing cellulosic shaped articles having inclusions of at least one nonpolar organic compound by the wet and dry extrusion process, **characterized in that**
a1) an emulsion comprising at least one nonpolar organic compound in a solution of cellulose in a solvent is prepared and stabilized by addition of at least one hydrophobic agent which increases the viscosity of the nonpolar organic compound, wherein the solvent employed is an ionic liquid, or
a2) for stabilization the emulsion is admixed with nanoscale sheetlike and/or elongate hydrophobized particles which surround the droplet-like inclusions of the nonpolar organic compound(s) and form a suspension and
b) the cellulose is recrystallized to obtain shaped articles having a cellulose matrix in which the nonpolar organic compound(s) is/are dispersedly incorporated,
wherein the cellulosic shaped articles are fibres and wherein the nanoscale sheetlike and/or elongate hydrophobized particles comprise modified phyllosilicates.

2. Process according to Claim 1, **characterized in that** nanotubes or nanofilaments are employed as the elongate particles.

3. Process according to Claim 1, **characterized in that** nanoscale-structured pyrogenic silica, polymers having olefinic and aromatic proportions, preferably styrene-butadiene block polymers, or phosphorus-containing esters are employed as the hydrophobic agent.

4. Process according to Claim 2, **characterized in that** hydrophobic agents are employed in a proportion of 1 % to 50 % by weight, preferably 1 % to 30 % by weight, based on the weight of the cellulose.

5. Process according to Claim 1, **characterized in that** both the hydrophobic agent(s) and the sheetlike nanoparticles are admixed with colorants, UV stabilizers, bactericidal substances, flame retardants, antistats, crosslinking agents, plasticizers and/or catalysts.

6. Process according to Claim 1, **characterized in that** hydrocarbons, waxes, beeswaxes, oils, fatty acids, fatty acid esters, stearic anhydrides, long-chain alcohols or mixtures thereof, each having a melting point of less than 100 °C, are employed as nonpolar organic compound(s).

7. Process according to Claim 1, **characterized in that** the nonpolar organic compound(s) is/are blended with other organic substances which lower the melting point of the nonpolar organic compounds.

8. Process according to claim 1, **characterized in that** the inclusions of the nonpolar organic compound(s) is/are obtained from solutions or suspensions of active ingredients, preferably nonpolar active ingredients in nonpolar organic solvents, wherein active ingredients employed are preferably plant-based products, such as jojoba oil, monoi oil, evening primrose oil, avocado oil, cocoa butter, essential plant extracts or nonpolar plant extracts, fat-soluble vitamins, such as vitamins A, D and E, and also insecticides, pyrethroids, preferably permethrin, or repellents, wherein the concentration of active ingredient(s) is 0.001 to 500 g or more per kg of shaped article.

9. Cellulosic shaped article producible by a process according to one or more of Claims 1 to 8, comprising a cellulose matrix and inclusions of the nonpolar organic compound(s) dispersed therein which comprises at least one hydrophobic, viscosity-increasing agent or in which the inclusions are surrounded by nanoscale sheetlike and/or elongate hydrophobized particles as a barrier material.

10. Cellulosic shaped article according to Claim 9, **characterized in that** the nonpolar organic compound(s) is/are selected from the group comprising hydrocarbons, waxes, beeswaxes, oils, fatty acids, fatty acid esters, stearic anhydrides and long-chain alcohols each having a melting point of less than 100 °C, or **in that** the proportion of the nonpolar organic compound(s) is more than 10 % by weight, preferably more than 30 % by weight and particularly preferably more than 40 % by weight based on the weight of the cellulose, wherein the nanoscale particles are preferably made of hydrophobized nanoscale pyrogenic silica.

11. Cellulosic shaped article according to Claim 9, **characterized in that** the inclusions contain one or more active ingredients from the group comprising plant-based products, jojoba oil, monoi oil, evening primrose oil, avocado oil, cocoa butter, essential plant extracts, nonpolar plant extracts, fat-soluble vitamins, vitamins A, D and E, insecticides, pyrethroids, permethrin and repellents, wherein the active ingredients are preferably present in an amount of up to 50% by weight based on the weight of the cellulosic shaped article.

12. Cellulosic shaped article according to Claim 9, **characterized in that** the proportion of the hydrophobic agent(s) is 1 % to 50 % by weight based on the weight of the cellulose.

13. Cellulosic shaped article according to Claims 9 and 11, **characterized in that** it contains a barrier material made of nanoscale particles for holding back the nonpolar organic compounds and for controlled release of the active ingredients, wherein the proportion of the barrier material is preferably 1 % to 50 % by weight based on the weight of the cellulose or wherein the barrier material preferably comprises nanoscale hydrophobized phyllosilicates, nanoscale hydrophobized bentonite or nanotubes or nanofilaments.

14. Cellulosic shaped article according to Claim 9, **characterized in that** the inclusions are surrounded by a zone having an elevated density of the barrier material.

15. Cellulosic shaped article according to Claim 9, **characterized in that** it is a fibre and **in that** according to a test according to DIN EN 26330 (1993) the loss of one or more nonpolar organic compounds after 20 washes is less than 20 % by weight based on the amount of the respective organic compound originally present in the fibre.

16. Cellulosic shaped article according to Claim 9, **characterized in that** it has a specific latent heat of greater than 20 J/g, preferably of greater than 30 J/g and particularly preferably of greater than 50 J/g in a temperature range of 15 °C to 45 °C.

17. Use of cellulosic shaped articles according to Claim 9 in textile fabrics, optionally blended with other textile fibres, for producing speciality papers.

## Revendications

1. Procédé pour la production de corps moulés en cellulose avec des inclusions d'au moins un composé organique non polaire selon le procédé d'extrusion par voie sèche/humide, **caractérisé en ce que**
a1) une émulsion est préparée avec au moins un composé organique non polaire dans une solution de cellulose dans un solvant et est stabilisée par l'ajout d'au moins un agent hydrophobe, qui augmente la viscosité du composé organique non polaire, dans lequel un liquide ionique est employé en tant que solvant,
ou
a2) sont ajoutées à l'émulsion pour la stabilisation des particules nanométriques, plates et/ou oblongues rendues hydrophobes qui entourent les inclusions de type gouttelettes du ou des composé(s) organique(s) non polaire(s) et forment une suspension, et
b) la cellulose est recristallisée, moyennant quoi des corps moulés sont obtenus avec une matrice de cellulose, dans laquelle le ou les composé(s) organique(s) non polaire(s) est/sont stocké(s) de manière dispersée,
dans lequel les corps moulés en cellulose sont des fibres et dans lequel les particules nanométriques, plates et/ou oblongues rendues hydrophobes comprennent des silicates en couches modifiés.

2. Procédé selon la revendication 1, **caractérisé en ce que** des nanotubes ou des nanofilaments sont employés par les particules oblongues.

3. Procédé selon la revendication 1, **caractérisé en ce que**, en tant qu'agent hydrophobe sont employés des polymères à structure nanométrique, de la silice pyrogène, avec des fractions oléfiniques et aromatiques, de préférence des polymères séquencés de styrène-butadiène ou des esters contenant du phosphore.

4. Procédé selon la revendication 2, **caractérisé en ce que** des agents hydrophobes avec une fraction de 1 à 50 % en poids, de préférence de 1 à 30 % en poids, par rapport au poids de la cellulose, sont employés.

5. Procédé selon la revendication 1, **caractérisé en ce que** sont ajoutés aussi bien à ou aux agent(s) hydrophobe(s) qu'aux nanoparticules plates des colorants, des stabilisateurs d'UV, des substances bactéricides, des agents ignifuges, des agents antistatiques, des agents de réticulation, des plastifiants et/ou des catalyseurs.

6. Procédé selon la revendication 1, **caractérisé en ce que** sont employés en tant que composés organiques non polaires des hydrocarbures, des cires, des cires d'abeilles, des huiles, des acides gras, des esters d'acides gras, des anhydrides stéariques, des alcools à chaîne longue ou des mélanges de ceux-ci, respectivement avec un point de fusion de moins de 100 °C.

7. Procédé selon la revendication 1, **caractérisé en ce que** le ou les composé(s) organique(s) non polaire(s) est/sont mélangé(s) avec d'autres substances organiques qui abaissent le point de fusion du ou des composés organiques non polaires.

8. Procédé selon la revendication 1, **caractérisé en ce que** les inclusions du ou des composés organiques non polaires sont obtenues à partir de solutions ou de suspensions de substances actives, de préférence des substances actives non polaires dans des solvants organiques non polaires, dans lequel sont employés en tant que substances actives de préférence des produits végétaux, comme l'huile de jojoba, l'huile de monoï, l'huile d'onagre, l'huile d'avocat, le beurre de cacao, des extraits végétaux éthérés ou des extraits végétaux non polaires, des vitamines liposolubles, comme la vitamine A, D et E, ainsi que des insecticides, des pyréthroïdes, de préférence la perméthrine, ou des agents répulsifs, dans lequel la concentration en substance(s) active(s) est de 0,001 à 500 g et plus par kg de corps moulé.

9. Corps moulé en cellulose, qui peut être produit selon un procédé selon l'une ou plusieurs des revendications 1 à 8, avec une matrice de cellulose et des inclusions qui y sont dispersées constituée par le ou les composé(s) organique(s) non polaire(s), qui contient au moins un agent augmentant la viscosité et hydrophobe ou dans lequel les inclusions sont entourées par des particules nanométriques plates et/ou oblongues rendues hydrophobes en tant que matériau formant barrière.

10. Corps moulé en cellulose selon la revendication 9, **caractérisé en ce que** le ou les composé(s) organique(s) non polaire(s) est/sont sélectionné(s) à partir du groupe comprenant des hydrocarbures, des cires, des cires d'abeilles, des huiles, des acides gras, des esters d'acides gras, des anhydrides stéariques et des alcools à chaîne longue, qui présentent respectivement un point de fusion de moins de 100 °C ou **en ce que** la fraction du ou des composé(s) organique(s) non polaire(s) est de plus de 10 % en poids, de préférence de plus de 30°% en poids et plus préférablement de plus de 40 % en poids, par rapport au poids de la cellulose, dans lequel les particules nanométriques sont de préférence composées de silice pyrogène nanométrique rendue hydrophobe.

11. Corps moulé en cellulose selon la revendication 9, **caractérisé en ce que** les inclusions contiennent une ou plusieurs substances actives issues du groupe comprenant des produits végétaux, de l'huile de jojoba, de l'huile de monoï, de l'huile d'onagre, de l'huile d'avocat, du beurre de cacao, des extraits végétaux éthérés, des extraits végétaux non polaires, des vitamines liposolubles, la vitamine A, D et E, ainsi que des insecticides, des pyréthroïdes, la perméthrine et des agents répulsifs, dans lequel les substances actives sont contenues de préférence en une quantité allant jusqu'à 50 % en poids, par rapport au poids du corps moulé en cellulose.

12. Corps moulé en cellulose selon la revendication 9, **caractérisé en ce que** la fraction du ou des agent(s) hydrophobe(s) est de 1 à 50% en poids par rapport au poids de la cellulose.

13. Corps moulé en cellulose selon les revendications 9 et 11, **caractérisé en ce qu'**il contient un matériau formant barrière constitué de particules nanométriques pour la conservation des composés organiques non polaires et pour la libération contrôlée des substances actives, dans lequel la fraction du matériau formant barrière est de 1 à 50% en poids, par rapport au poids de la cellulose, ou dans lequel le matériau formant barrière comprend de préférence des silicates en couches rendus hydrophobes nanométriques, de la bentonite rendue hydrophobe nanométrique ou des nanotubes ou des nanofilaments.

14. Corps moulé en cellulose selon la revendication 9, **caractérisé en ce que** les inclusions sont entourées par une zone avec une densité plus élevée du matériau formant barrière.

15. Corps moulé en cellulose selon la revendication 9, **caractérisé en ce qu'**il s'agit d'une fibre et **en ce que** selon un test selon la norme DIN EN 26330 (1993) la perte d'un ou plusieurs composés organiques non polaires après 20 lavages est de moins de 20% en poids, par rapport à la quantité du composé organique respectif contenue à l'origine dans la fibre.

16. Corps moulé en cellulose selon la revendication 9, **caractérisé en ce qu'**il présente dans une plage de températures de 15 à 45 °C une chaleur latente spécifique de plus de 20 J/g, de préférence de plus de 30 J/g et plus préférablement de plus de 50 J/g.

17. Utilisation de corps moulés en cellulose selon la revendication 9 dans des structures planes textiles, le cas échéant par mélange avec d'autres fibres textiles, pour la production de papiers spéciaux.
